# EUROPÄISCHE PATENTSCHRIFT

(11) **EP 0 358 074 B1**
(45) Veröffentlichungstag und Bekanntmachung des Hinweises auf die Patenterteilung: **05.01.1994**
(21) Anmeldenummer: 89115802.4
(22) Anmeldetag: 26.08.1989
(51) Int. Cl.: C07C 69/90, C07C 67/14, C08F 222/14

(54) **Neue Bisester ungesättigter Carbonsäuren, ihre Herstellung und Verwendung in härtbaren Gemischen**
Biesters of unsaturated carboxylic acids, their preparation and use in curable compositions
Biesters d'acides carboxyliques insaturés, leur préparation et utilisation dans des mélanges durcissables

(30) Priorität: 08.09.1988 DE 3830453
(43) Veröffentlichungstag der Anmeldung: 14.03.1990
(73) Patentinhaber: BAYER AG, 51368 Leverkusen (DE)
(72) Erfinder: Meier, Helmut-Martin, Dr., D-4030 Ratingen 6 (DE); Pielartzik, Harald, Dr., D-4150 Krefeld (DE); Morbitzer, Leo, Dr., D-5000 Köln 80 (DE); Müller, Hanns-Peter, Dr., D-5060 Bergisch-Gladbach 2 (DE); Braun, Dietrich, Prof. Dr., D-6100 Darmstadt-Arheillgen (DE)

(56) Entgegenhaltungen:
- US-A- 4 452 993

## Beschreibung

Die Erfindung betrifft neue Bisester ungesättigter Carbonsäuren, ein Verfahren zu ihrer Herstellung sowie ihre Verwendung in härtbaren Gemischen.

Ungesättigte Bisester, z.B. Bisacrylate von Diphenolen, sind als Ausgangsprodukte zur Herstellung von vernetzten Formkörpern bekannt (vgl. EP 0 069 069). Die Verwendung abriebbeständiger Acrylatharze auf dem Dentalgebiet ist aus JA 79/001 400 bekannt. Die in der japanischen Patentveröffentlichung beschriebenen Bis-(meth)-acrylate werden in SU 560 891 als Comonomere für die Herstellung von Beleuchtungskörpern beschrieben. Weitere, flüssigkristalline Acrylate bzw. Bisacrylate und Bismethacrylate sind beispielsweise beschrieben von H. Finkelmann in Adv. Polym. Sci. 59/60. In der US-PS 4 683 327 werden thermisch härtbare, anisotrope Acrylpolymere aus Hydroxyethoxyphenol, Terephthaloylchlorid und (Meth)Acrylchlorid beschrieben. Außerdem sind bestimmte flüssigkristalline Bisacrylate aus Makromol. Chem. 186, 977 (1985) zur Herstellung von Polymeren bekannt.

Gegenstand der Erfindung sind Bisester von ungesättigten Carbonsäuren der Formel (I) 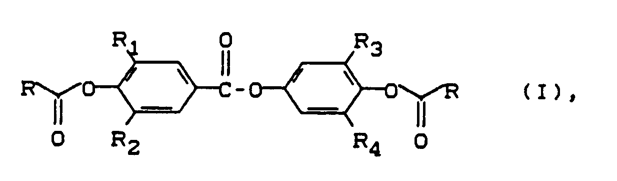
worin
- R₁, R₂, R₃, R₄: unabhängig voneinander Wasserstoff, Halogen oder Alkyl mit 1 bis 6 C-Atomen, vorzugsweise 1 bis 4 C-Atomen und
- R: Reste von ungesättigten Carbonsäuren bedeuten.

Als ungesättigte Carbonsäuren kommen in Betracht: Acrylsäure, Methacrylsäure, 2-Butensäure, 3-Methyl-2-butensäure, Undecylensäure, Ölsäure, Sorbinsäure, Linolsäure) vorzugsweise Acrylsäure, Methacrylsäure und 2-Butensäure.

Als Halogene seien genannt: Fluor, Chlor, Brom, vorzugsweise Chlor.

Als Alkylgruppen kommen in Frage: die Methyl-, Ethyl-, Propyl-, Butyl-, Pentyl- und Hexylgruppe sowie deren Isomeren, bevorzugt Methyl, Ethyl und Propyl.

Besonders bevorzugt sind Verbindungen der Formel (I), worin R¹, R², R³ und R⁴ unabhängig voneinander Wasserstoff, Chlor, Brom oder Alkyl mit 1 bis 4 C-Atomen, wie Methyl Ethyl und Propyl, bedeuten und R sich von Acryl-, Methacryl-und 2-Butensäure ableitet.

Ganz besonders bevorzugt sind solche Verbindungen der Formel (I), in denen R¹, R², R³ und R⁴ Wasserstoff bebedeutet und R sich von Acrylsäure ableitet.

Gegenstand der Erfindung ist auch ein Verfahren zur Herstellung der Bisester ungesättigter Carbonsäuren der Formel (I) 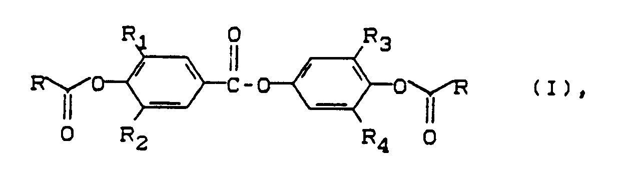
worin
- R₁, R₂, R₃, R₄: unabhängig voneinander Wasserstoff, Halogen oder Alkyl mit 1 bis 6 C-Atomen, vorzugsweise 1 bis 4 C-Atomen, und
- R: Reste von ungesättigten Carbonsäuren bedeuten,
das dadurch gekennzeichnet ist, daß man Bisphenole der Formel (II) 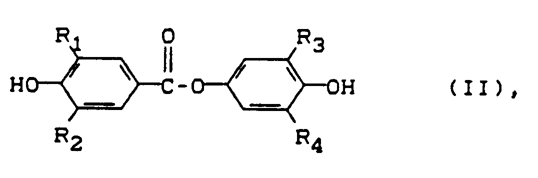
worin
- R₁, R₂, R₃ und R₄: die Bedeutung wie in Formel (I) haben,
mit ungesättigten Säuren oder deren reaktionsfähigen Derivate in Gegenwart von Katalysatoren und gegebenenfalls Basen umsetzt.

Als ungesättigte Carbonsäuren kommen die zuvor genannten in Frage.

Die Umsetzung geschieht dabei in bekannter Weise wie in Houben-Weyl, Methoden der Organischen Chemie, Carbonsäuren und Carbonsäurederivate, Ergänzungsband E 5/Teil 1, beschrieben. Dort werden auch reaktionsfähige Säurederivate bzw. aktivierte Säuren oder deren Derivate beschrieben.

Vorzugsweise werden die erfindungsgemäßen Bisester ungesättigter Carbonsäuren aus dem entsprechenden Bisphenol und den entsprechenden Säurehalogeniden nach einer Schotten-Baumann-Reaktion oder nach dem Phasengrenzflächenverfahren hergestellt.

Als Katalysatoren werden bei der Umsetzung bevorzugt tert. Amine, wie Tributylamin und/oder Triethylenamin, sowie Ammoniumsalze, wie Triethylbenzylammonium oder Tetrabutylammoniumchlorid und -bromid eingesetzt.

Katalysatoren für die Phasengrenzflächenreaktion sind dem Fachmann bekannt und beschrieben z.B. im "Compendium of Phase-Transfer Reactions and Related Synthetic Methods, 1979 Fluka AG.

Als Basenfänger können bei der Schotten-Baumann-Reaktion Triethylamin, Diisopropylamin, Pyridin, 2,6-Dimethylpyridin, N,N-Dimethylanilin, 1,8-Bis-(dimethylamino)naphthalin und/oder Diazabicyclo-(5.4.0)-undec-11-en eingesetzt werden.

Die Umsetzung wird vorzugsweise in Lösungsmitteln wie Diethylether, Diisopropylether, Dioxan, Tetrahydrofuran, halogenierten Kohlenwasserstoffen wie Methylenchlorid, Chloroform, 1,2-Dichlorether und/oder Trichlorether, durchgeführt.

Die Herstellung der Bisphenole der Formel (II) mit R₁ bis R₄ für Wasserstoff wird beispielsweise beschrieben in der EP 252 357.

Ein weiterer Gegenstand der Erfindung ist die Verwendung der neuen Bisester ungesättigter Carbonsäuren in härtbaren Polyesterharzmischungen, die üblicherweise zur Herstellung von Formkörpern eingesetzt werden können.

Ein weiterer Gegenstand der Erfindung sind daher härtbare Polyesterharzmischungen, enthaltend die erfindungsgemäßen Bisester ungesättigter Carbonsäuren der Formel (I) in Mengen von ca. 5 bis 95 Gew.-%, bevorzugt 10 bis 50 Gew.-%, sowie ungesättigte Polyester in Mengen von 95 bis 5 Gew.-%, bevorzugt 90 bis 50 Gew.-%.

Ungesättigte Polyesterharze im Sinne der Erfindung bestehen aus
A) 30 bis 35, vorzugsweise 50 bis 90, Gew.-Teilen ungesättigtem Polyester und
B) 70 bis 5, vorzugsweise 40 bis 10, Gew.-Teilen mit A) copolymerisierbarem Monomer.

Die verwendbaren ungesättigten Polyester und die mit den ungesättigten Polyestern copolymerisierbaren Monomere sind bekannt und beispielsweise beschrieben in J. Björksten et al. "Polyesters and their Applications", Reinhold Publishing Corp., New York 1956, J.R. Lawrence "Polyester Resins", Reinhold Publ. Corp., New York 1960, S. 18f., Kunststoff-Handbuch Bd. VIII ("Polyester"), Carl Hanser Verlag, München 1973, S. 247-312 sowie in der DE-AS 1 024 654.

Die aus den erfindungsgemäßen Bisester ungesättigter Carbonsäuren hergestellten härtbaren Mischungen besitzen folgende Vorteile:
Sie zeigen einen höheren Glasübergang als gewöhnliche Polyesterharze und weisen, trotz höherer Netzbogendichte, eine weitmaschigere Vernetzung auf. Dies ist überraschend, da normalerweise eine hohe Netzbogendichte mit einer engmaschigen Vernetzung korreliert.

### Beispiele

### Beispiel 1

115,1 g (0,5 Mol) 4-Hydroxyphenyl-p-hydroxybenzoat, 111,3 g (1,1 Mol) Triethylamin in 500 ml Dioxan werden bei Raumtemperatur tropfenweise mit 99,6 g (1,1 Mol) Acrylsäurechlorid versetzt (ca. 2 Stdn.). Die Reaktionsmischung wird abgesaugt und mit kaltem Dioxan gewaschen. Die Mutterlauge wurde im Wasserstrahlvakuum eingeengt, der Rückstand in Methylenchlorid gelöst und mit Wasser neutral gewaschen, über Na₂SO₄ getrocknet, filtriert und eingeengt. Der Rückstand wird aus Ethanol umkristallisiert.
Ausbeute: 100 g gelb-beiger Feststoff
Fp.: 99°C
LC-Bereich: 99-147°C 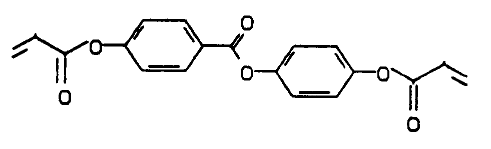
- ¹H-NMR:: 8,26-7,27 m, 8H; 6,61, dd, 2H; 6,3, m, 2H; 6,0, dd

### Beispiel 2

In einem im Vakuum ausgeheizten und mit Stickstoff belüfteten 500 ml-Dreihalskolben mit Tropftrichter, Innenthermometer und Rückflußkühler werden 23,01 g (0,1 Mol) 4-Hydroxybenzoesäure-4′-hydroxyphenylester, 20,74 g (0,205 Mol) Triethylamin und 0,2 g 2,6-Di-tert.-butyl-4-methoxyphenol in 200 ml trockenem Chloroform gelöst. Anschließend werden 20,9 g (0,2 Mol) frisch destilliertes Methacrylsäurechlorid in 20 ml trockenem Chloroform so zugetropft, daß sich eine Innentemperatur von 25-28°C einstellt. Es wird über Nacht bei Raumtemperatur nachgerührt, dann mit destilliertem Wasser chloridfrei gewaschen, die organische Phase abgetrennt, mit Na₂SO₄ getrocknet und eingeengt. Der verbleibende Rückstand wird aus Methanol umkristallisiert und bei Raumtemperatur im Wasserstrahlvakuum getrocknet.
Fp.: 149-151°C;
Ausbeute: 73 %
- ¹H-NMR:: 8,26 bis 7,17, Multiplett, 8 arom. Protonen; 6,39, 6,36, 5,81 und 5,77, 4 vinylische Protonen; 2,07 und 2,08, Singulett, 6 aliphatische Protonen.

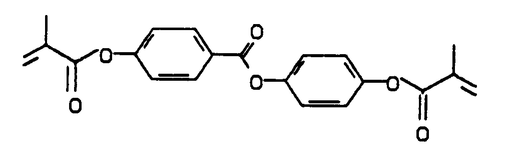

### Verwendungsbeispiel

Aus 1514 g 1,2-Propylenglykol, 810 g Dipropylenglykol, 1919 g Maleinsäureanhydrid und 588 g Phthalsäureanhydrid wird in der üblichen Schmelzkondensation bei 210°C unter Stickstoff ein ungesättigtes Polyesterharz hergestellt, das in Styrol gelöst folgende Kennzahlen besitzt:
Festgehalt: 80 %
Säurezahl: 20-22 mg KOH/g
Farbzahl: ca. 3
Inhibitor: 0,02 % Hydrochinon-Paste
Viskosität: 60 000 mPas

Zu diesem ungesättigten Polyesterharz gibt man 6,4 % Styrol und 11,3 % 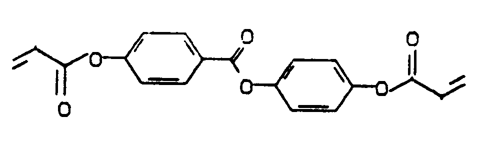
sowie 1 % Benzoylperoxid und härtet die Mischung zu einer 4 mm dicken Platte bei 80°C aus. Die Platte wird 65 Std. bei 80°C getempert. Der Formkörper zeigt ein opakes Aussehen. Er wird aus F 1 bezeichnet.

Aus dem obigen Stammharz wird im Vergleich durch Zugabe von 13,4 % Styrol ein 2. Formkörper analog hergestellt, aber ohne Verwendung der flüssigkristallinen Komponente. Er wird als F 2 bezeichnet. F 2 zeigt ein durchscheinendes Aussehen.

Aus Modulmessungen sind unterschiedliche Ergebnisse für F 1 und F 2 zu erkennen. F 1 zeigt einen weniger breiten aber höheren Glasübergang bei 57°C als F 2, der einen breiten aber etwas niedrigeren Glasübergang aufweist.

Oberhalb des Hauptglasübergangs (75-225°C) deuten die unterschiedlichen Modulverläufe auf eine unterschiedliche Vernetzungsstruktur hin. In gewissen Bereichen zeigt F 1 eine weitmaschigere Vernetzung als F 2; dies ist überraschend, obwohl die Netzbogendichte von F 1 höher als die von F 2 ist.

## Patentansprüche

1. Bisester ungesättigter Carbonsäuren der Formel 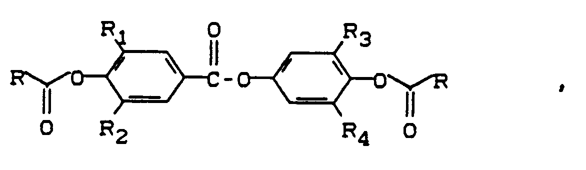 worin
R₁, R₂, R₃ und R₄ unabhängig voneinander Wasserstoff, Halogen oder Alkyl mit 1 bis 6 C-Atomen und
R Reste von ungesättigten Carbonsäuren bedeuten.

2. Verfahren zur Herstellung der Bisester der Formel 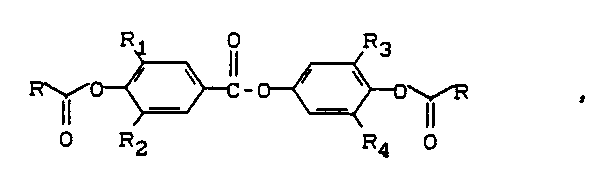 worin
R₁, R₂, R₃ und R₄ unabhängig voneinander Wasserstoff, Halogen oder Alkyl mit 1 bis 6 C-Atomen und
R Reste von ungesättigten Carbonsäuren bedeuten,
dadurch gekennzeichnet, daß man Bisphenole der Formel 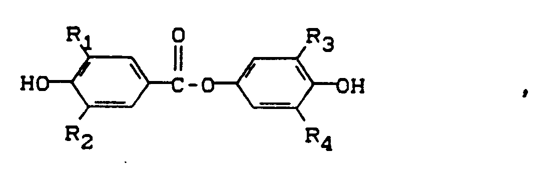 worin
R₁ bis R₄ die oben angegebene Bedeutung besitzen,
mit reaktiven, ungesättigten Carbonsäurederivaten in Gegenwart von Katalysatoren und gegebenenfalls Basen umsetzt.

3. Verwendung der Bisester ungesättigter Carbonsäuren nach Anspruch 1 in härtbaren Polyesterharzmischungen.

4. Härtbare Polyesterharzmischungen, enthaltend 5 bis 95 Gew.-% an Bisester ungesättigter Carbonsäuren nach Anspruch 1 und 95 bis 5 Gew.-% ungesättigtes Polyesterharz, das zu 30 bis 95 Gew.-Teilen aus ungesättigtem Polyester und zu 70 bis 5 Gew.-Teilen aus mit dem ungesättigten Polyester copolymerisierbaren Monomeren besteht.

## Claims

1. Bisesters of unsaturated carboxylic acids corresponding to the following formula: 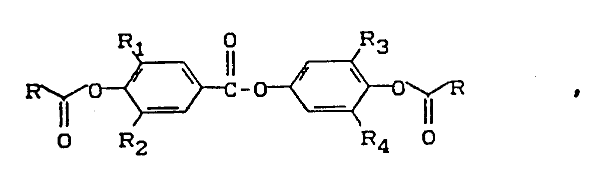 in which
R₁, R₂, R₃ and R₄ independently of one another represent hydrogen, halogen or C₁₋₆ alkyl and
R represents residues of unsaturated carboxylic acids.

2. A process for the production of the bisesters corresponding to the formula: 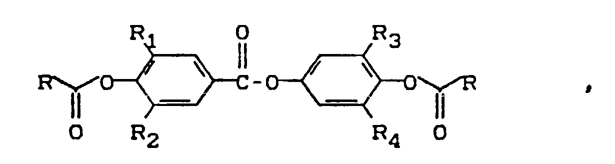 in which
R₁, R₂, R₃ and R₄ independently of one another represent hydrogen, halogen or C₁₋₆ alkyl and
R represents residues of unsaturated carboxylic acids,
characterized in that bisphenols corresponding to the following formula: 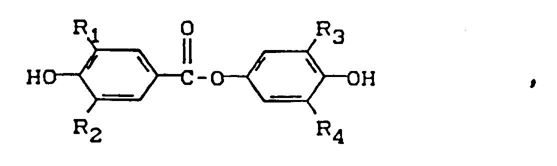 in which
R₁ to R₄ are as defined above,
are reacted with reactive unsaturated carboxylic acid derivatives in the presence of catalysts and, optionally, bases.

3. The use of the bisesters of unsaturated carboxylic acids claimed in claim 1 in curable polyester resin mixtures.

4. Curable polyester resin mixtures containing 5 to 95% by weight of the bisesters of unsaturated carboxylic acids claimed in claim 1 and 95 to 5% by weight unsaturated polyester resin of which 30 to 95 parts by weight consist of unsaturated polyester and 70 to 5 parts by weight of monomers copolymerizable with the unsaturated polyester.

## Revendications

1. Bis-esters d'acides carboxyliques insaturés de formule : 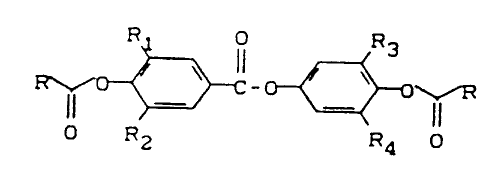 dans laquelle
R₁, R₂, R₃, R₄ représentent chacun, indépendamment les uns des autres, l'hydrogène, un halogène ou un groupe alkyle en C₁-C₆, et
les symboles R représentent des radicaux d'acides carboxyliques insaturés.

2. Procédé de préparation des bis-esters de formule : 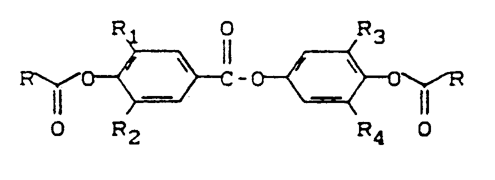 dans laquelle
R₁, R₂, R₃, R₄ représentent chacun, indépendamment les uns des autres, l'hydrogène, un halogène ou un groupe alkyle en C₁-C₆ et
les symboles R représentent des radicaux d'acides carboxyliques insaturés, caractérisé en ce que l'on fait réagir des bisphénols de formule : 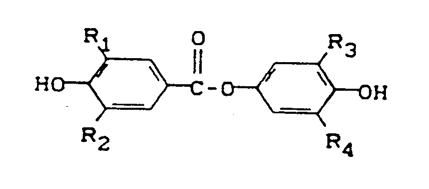 dans laquelle
R₁ à R₄ ont les significations indiquées ci-dessus,
avec des dérivés réactifs d'acides carboxyliques insaturés en présence de catalyseurs et le cas échéant de bases.

3. Utilisation des bis-esters d'acides carboxyliques insaturés selon revendication 1 dans des mélanges durcissables à base de résines de polyesters.

4. Mélanges durcissables à base de résines de polyesters, contenant 5 à 95 % en poids de bis-esters d'acides carboxyliques insaturés selon revendication 1 et 95 à 5 % en poids d'une résine de polyester insaturé consistant elle-même en 30 à 95 parties en poids d'un polyester insaturé et 70 à 5 parties en poids de monomères copolymérisables avec le polyester insaturé.
